(19) **Europäisches Patentamt**
**European Patent Office**
**Office européen des brevets**

(11) **EP 1 884 184 B1**

(12) **EUROPEAN PATENT SPECIFICATION**

(45) Date of publication and mention
of the grant of the patent:
**01.02.2012 Bulletin 2012/05**

(51) Int Cl.:
*A61B 1/04* (2006.01)     *A61B 1/05* (2006.01)
*A61B 5/06* (2006.01)     *A61B 5/07* (2006.01)
*G01S 5/02* (2010.01)

(21) Application number: **06745527.9**

(22) Date of filing: **20.04.2006**

(86) International application number:
**PCT/JP2006/308346**

(87) International publication number:
**WO 2006/126350 (30.11.2006 Gazette 2006/48)**

(54) **CAPSULE MEDICAL SYSTEM**

MEDIZINISCHES KAPSELSYSTEM

SYSTEME MEDICAL DE CAPSULE

(84) Designated Contracting States:
**DE FR GB**

(30) Priority: **26.05.2005 JP 2005154371**
**24.01.2006 JP 2006015612**

(43) Date of publication of application:
**06.02.2008 Bulletin 2008/06**

(73) Proprietor: **OLYMPUS MEDICAL SYSTEMS CORP.**
**Tokyo 151-0072 (JP)**

(72) Inventors:
• **HASEGAWA, Jun**
**Olympus Medical Systems Corp.**
**Tokyo151-0072 (JP)**

• **NONAMI, Tetsuo,**
**Olympus Medical Systems Corp.**
**Tokyo 151-0072 (JP)**

(74) Representative: **von Hellfeld, Axel**
**Wuesthoff & Wuesthoff**
**Patent- und Rechtsanwälte**
**Schweigerstrasse 2**
**81541 München (DE)**

(56) References cited:
**EP-A- 0 667 115        EP-A- 1 260 176**
**WO-A-2005/067781    JP-A- 11 325 810**
**JP-A- 2001 046 357    JP-A- 2002 236 166**
**JP-A- 2004 041 709**

EP 1 884 184 B1

## Description

Technical Field

The present invention relates to a capsule medical system that is placed within a living body so as to, for example, capture an image.

Background Art

Endoscopes have been in widespread use in medical field and etc. by inserting an insertion portion of the endoscope into a body cavity of a patient in order to examine or treat the patient as needed.

In recent years, swallowable capsule medical systems have been available that capture an image of the interior of the body cavity for inspection of the body cavity.

In general, when the capsule medical system is placed in the body cavity, the capsule medical system moves in the body cavity due to the peristaltic motion. Accordingly, a user may want to know at which location in the body cavity the information from the capsule medical system is obtained.

To address this issue, Japanese Unexamined Patent Application Publication No. 2003-135389, for example, describes a technology in which a plurality of antennas disposed outside the human body receive a radio signal transmitted from an antenna of the capsule inside the body cavity so as to compute the location of the capsule on the basis of the strength of the received signal.

In addition, Japanese Patent No. 3571675 describes an apparatus and a method in which a plurality of source coils are disposed in the insertion portion of an endoscope in the lengthwise direction thereof so as to detect a magnetic field generated by the source coils using a plurality of coils disposed outside the human body. Thus, the location and orientation of the insertion portion of an endoscope can be detected.

However, Japanese Unexamined Patent Application Publication No. 2003-135389 describes neither a method of specifically detecting the location of the capsule nor a method of detecting the orientation of the capsule or the antenna in the capsule from the transmitted radio signal.

In addition, the method disclosed in Japanese Patent No. 3571675 is used for detecting the shape of an insertion portion in a case of an endoscope including an insertion portion inserted into a body cavity, so that the method is not the one applied to an endoscopic capsule.

WO 2005/067781 A1 discloses a capsule medical system comprising a capsule endoscope which is adapted to be swallowed by a patient. The capsule endoscope is provided with an antenna and a communication processing circuit for transmitting image signals. The system further comprises a plurality of antennas adapted to be disposed outside the body of the patient. A program for estimating the position and orientation of the capsule endoscope on the basis of the strength of signals received through the extracorporal antennas is installed in a signal processing circuit of an external device. The signal processing circuit obtains twelve nonlinear equations, in which the positions and orientations of single-core coils arranged in the capsule endoscope are unknown. These nonlinear equations are solved by iterative refinement.

The present invention is conceived in view of the above-described points and an object of the present invention is to provide a capsule medical system capable of accurately detecting a location and/or orientation at which biological information such as an image captured in a living body, is acquired.

Disclosure of Invention

The above defined problem is solved by a capsule medical system comprising the features defined in claim 1.

Means for Solving the Problem

According to an embodiment of the present invention, a capsule medical system includes a capsule intracorporeal device placed in a living body, where the capsule intracorporeal device includes an antenna, wireless transmitting means for wirelessly transmitting an electromagnetic wave signal from the antenna of the capsule intracorporeal device, a plurality of extracorporeal antennas disposed outside the living body, estimating means for estimating at least one of the location and the orientation of the antenna on the basis of the electromagnetic wave signal received by the plurality of extracorporeal antennas, and updating and correcting means. The updating and correcting means compares an estimated value computed using the at least one of the estimated location and the estimated orientation with the actually detected value and repeatedly updates and corrects the at least one of the location and the orientation estimated by the estimating means until an update value for the at least one of the location and the orientation computed on the basis of the compared values is less than or equal to a predetermined value, wherein the estimating means estimates the location of the antenna on the basis of the electromagnetic wave signal received by the plurality of extracorporeal antennas using a theoretical formula that takes into account the attenuation of the electromagnetic wave signal in the living body.

In such a structure, by repeatedly performing a correction process in which the estimated value of the location and/or orientation of the antenna incorporated in the capsule intracorporeal device is updated, the location and/or orientation of the antenna can be accurately estimated.

Brief Description of the Drawings

Fig. 1A illustrates an exemplary structure of the main part of a capsule medical system according to a first exemplary embodiment of the present invention;

Fig. 1B illustrates an extracorporeal device shown in Fig. 1A that is connected to a data station via a cradle;

Fig. 2 illustrates the internal structure of a capsule endoscope shown in Fig. 1A;

Fig. 3 illustrates an exemplary arrangement of a plurality of antennas of an antenna unit shown in Fig. 1A and the coordinate system defined for the antennas;

Fig. 4 is a block diagram schematically illustrating the internal structure of the capsule endoscope system shown in Fig. 1A;

Fig. 5A illustrates an example of a signal transmitted from the capsule endoscope shown in Fig. 1 during a frame period;

Fig. 5B illustrates another example of the signal transmitted from the capsule endoscope shown in Fig. 1 during a frame period;

Fig. 6 is a diagram illustrating a component of the electromagnetic field at a given point P when the location of the antenna shown in Fig. 2 is defined as the origin;

Fig. 7 is a diagram illustrating a component of an electric field shown in Fig. 6 using a component of the Cartesian coordinate system;

Fig. 8 is a diagram illustrating the attenuation of electromagnetic waves when propagating in a medium;

Fig. 9 is a diagram illustrating an electromotive force detected by a bar antenna attached to the surface of a human body when the bar antenna receives an electric field generated by the antenna shown in Fig. 6;

Fig. 10 is a flow diagram illustrating the procedure of an estimation process of the location and orientation of the antenna shown in Fig. 2;

Fig. 11A illustrates an example of display when an image captured by the capsule endoscope shown in Fig. 1A and the trajectory of the estimated location are simultaneously displayed;

Fig. 11B illustrates another example of display when an image captured by the capsule endoscope shown in Fig. 1A and the trajectory of the estimated location are simultaneously displayed;

Fig. 12 illustrates the shape of an antenna used in an antenna unit according to a third exemplary embodiment of the present invention;

Fig. 13 is a schematic illustration of the internal structure of a capsule endoscope according to a second modification of the third exemplary embodiment;

Fig. 14A illustrates an example of a signal transmitted from the capsule endoscope shown in Fig. 13; and

Fig. 14B illustrates another example of the signal transmitted from the capsule endoscope shown in Fig. 13.

Best Mode for Carrying Out the Invention

Embodiments of the present invention are described with reference to the accompanying drawings.

First Embodiment

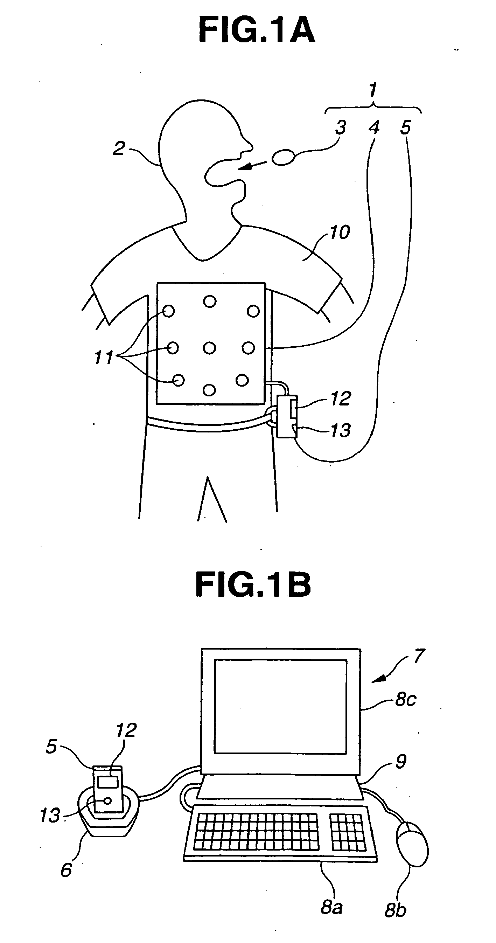

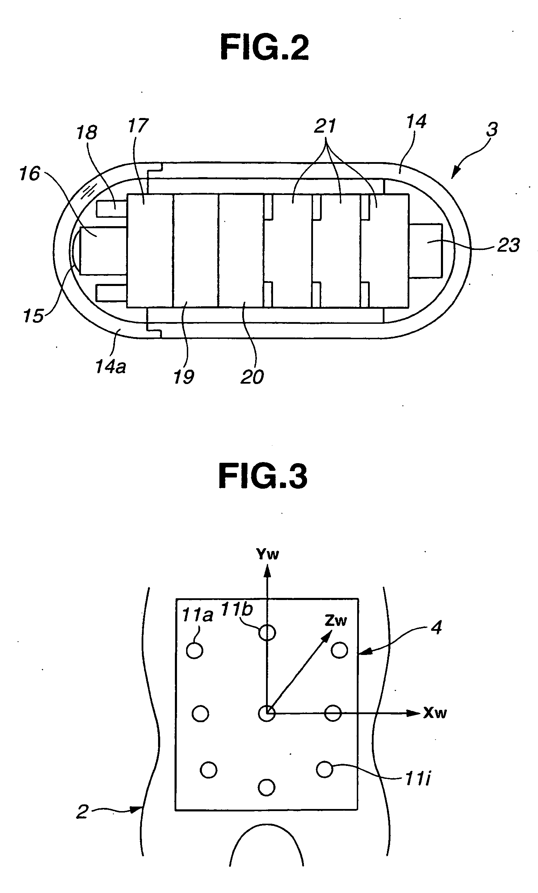

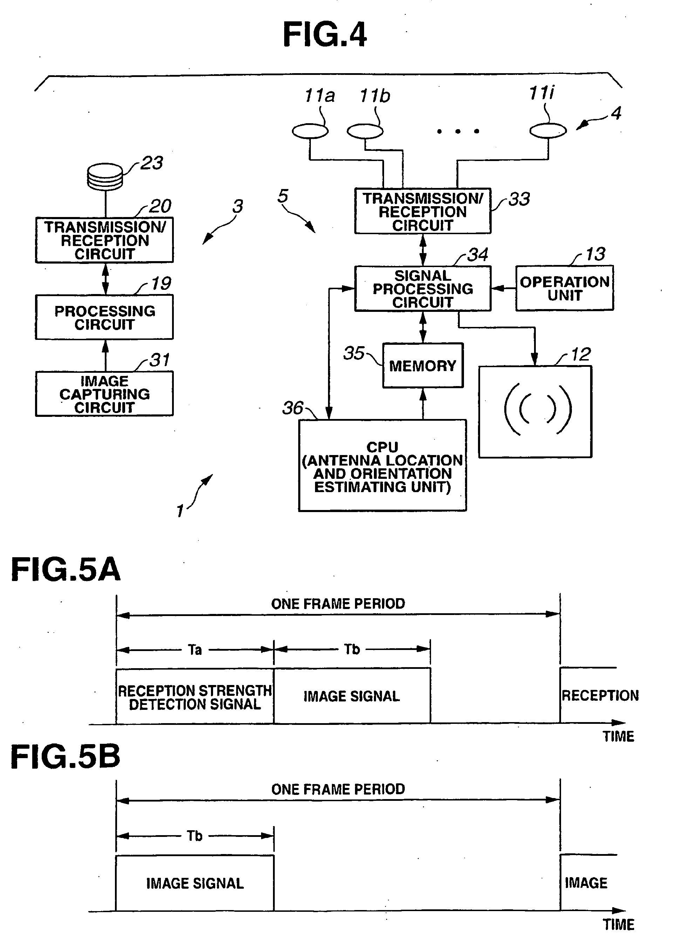

Figs. 1A to 11 relate to a first embodiment of the present invention. Fig. 1A illustrates an exemplary main portion of a capsule medical system according to the first embodiment of the present invention. Fig. 1B illustrates an extracorporeal device shown in Fig. 1A that is connected to a data station via a cradle. Fig. 2 illustrates the internal structure of a capsule endoscope. Fig. 3 illustrates an exemplary arrangement of a plurality of antennas of an antenna unit and the coordinate system defined for the antennas. Fig. 4 is a block diagram schematically illustrating the internal structure of the capsule endoscope system shown in Fig. 1A .

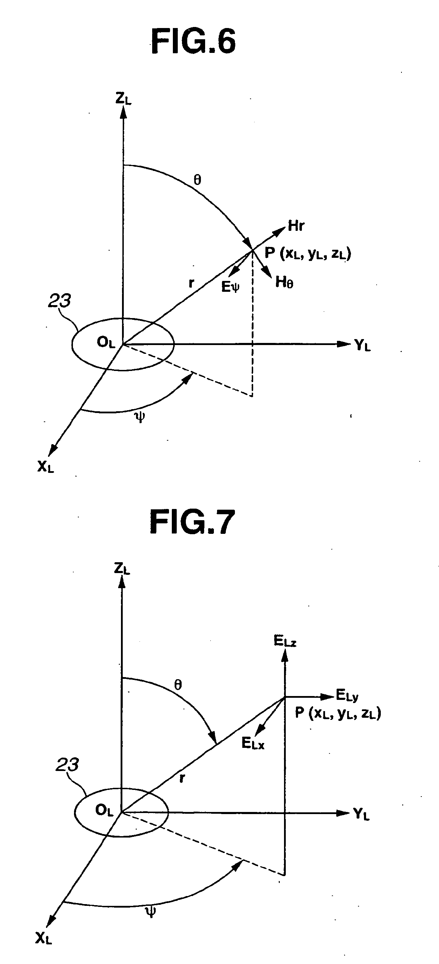

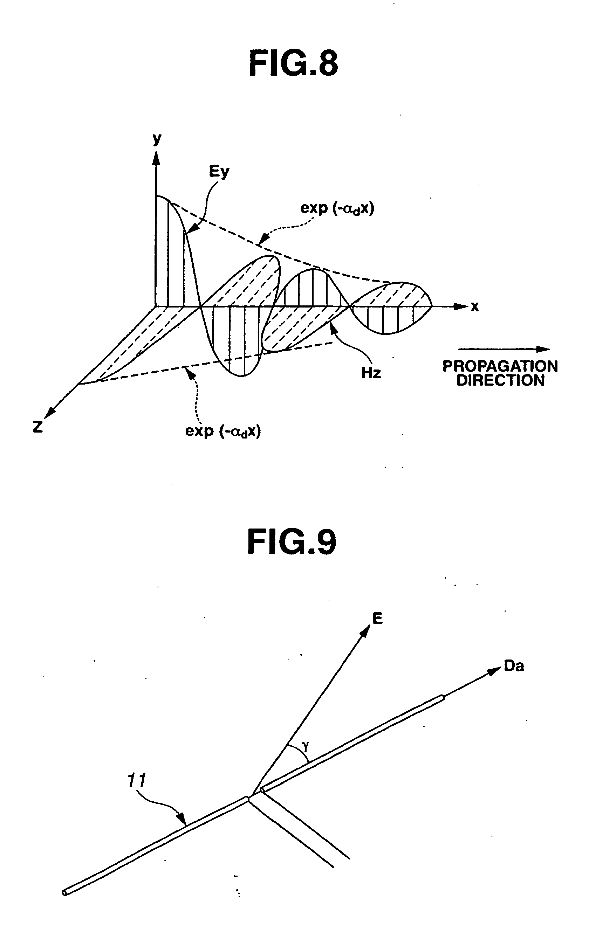

Fig. 5A illustrates an example of a signal transmitted from the capsule endoscope shown in Fig. 1A during a frame period. Fig. 5B illustrates another example of the signal transmitted from the capsule endoscope shown in Fig. 1A during a frame period. Fig. 6 is a diagram illustrating a component of an electromagnetic field at a given point P when the location of the antenna shown in Fig. 2 is defined as the origin. Fig. 7 is a diagram illustrating a component of an electric field shown in Fig. 6 using a component of the Cartesian coordinate system. Fig. 8 is a diagram illustrating the attenuation of electromagnetic waves when the electromagnetic waves propagate in a medium. Fig. 9 is a diagram illustrating an electromotive force detected by a bar antenna attached to the surface of a human body when the bar antenna receives an electric field generated by the antenna shown in Fig. 6.

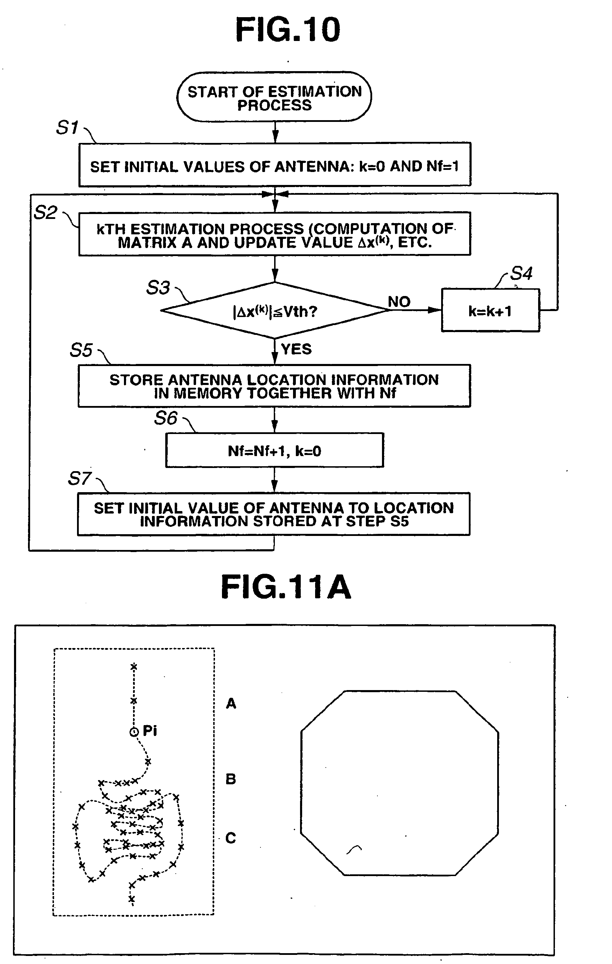

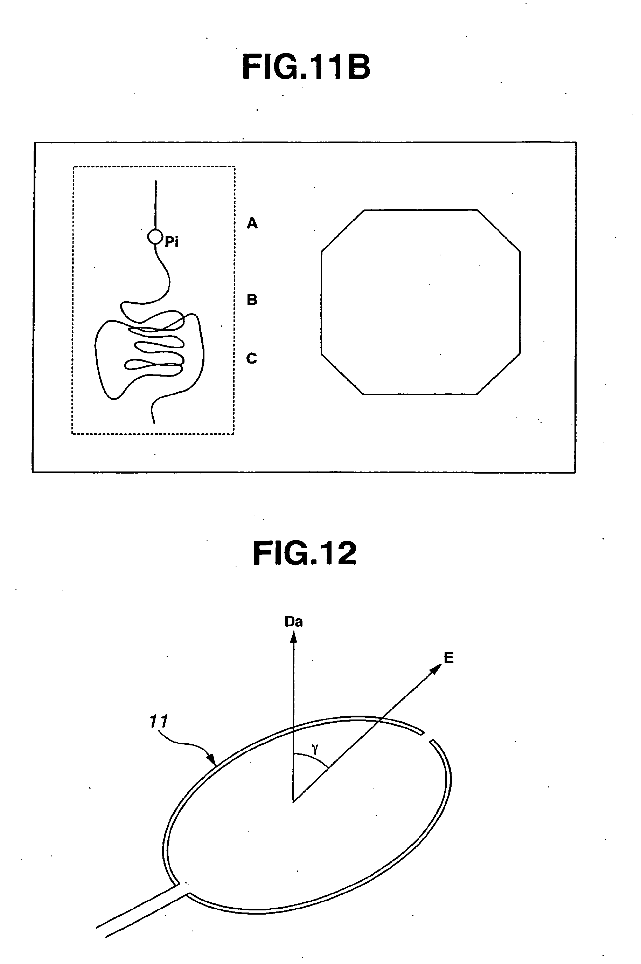

Fig. 10 is a flow diagram illustrating the procedure of an estimation process of the location and orientation of the antenna shown in Fig. 2. Fig. 11A illustrates an example of a display screen when an image captured by the capsule endoscope shown in Fig. 1A and the trajectory of the estimated location are simultaneously displayed. Fig. 11B illustrates another example of a display screen when an image captured by the capsule endoscope shown in Fig. 1A and the trajectory of the estimated location are simultaneously displayed.

According to the first embodiment of the present invention, as shown in Fig. 1A, a capsule endoscope system 1 includes as main parts an endoscopic capsule 3 that a patient 2 swallows, an antenna unit 4 disposed outside the body of the patient 2, and an extracorporeal device (or an external device) 5 connected to the antenna unit 4. The endoscopic capsule 3 functions as a capsule intracorporeal device for examining the body cavity of the patient 2. The antenna unit 4 wirelessly receives information on an image captured by the endoscopic capsule 3.

As shown in Fig. 1B , when mounted on a cradle 6, the extracorporeal device 5 is electrically connected to a data station 7, such as a personal computer. The data station 7 can retrieve images stored in the extracorporeal device 5 when operated using an input/operating device, such as a keyboard 8a and a mouse 8b. Subsequently, the data station 7 can display the retrieved image on a monitor unit 8c.

As shown in Fig. 1A, the patient 2 wears a jacket 10 in order to undergo endoscopic examination after the patient 2 swallows the endoscopic capsule 3. The jacket 10 includes the antenna unit 4 including a plurality of antennas 11.

The endoscopic capsule 3 captures an image and transmits the signal of the image via an antenna 23 incorporated in the endoscopic capsule 3 (see Fig. 2). The plurality of antennas 11 of the antenna unit 4 receive the transmitted signal. The extracorporeal device 5 that is connected to the antenna unit 4 can store the image captured by the endoscopic capsule 3.

The extracorporeal device 5 has, for example, a box shape. A liquid crystal monitor 12 for displaying an image and an operation unit 13 for inputting instruction of the operation are provided on the front surface of the extracorporeal device 5.

The extracorporeal device 5 may include only an alarm LED for indicating a battery level and a power switch functioning as the operation unit 13. Additionally, as a secondary extracorporeal device, a portable display unit (a viewer) (not shown) may be connected to the extracorporeal device 5 so as to process the image signal transmitted from the endoscopic capsule 3 and display the image on a built-in liquid crystal monitor.

As shown in Fig. 2, the endoscopic capsule 3 includes a casing 14 having a cylindrical shape with a closed rear end and a substantially dome-shaped cover 14a coupled with a front end of the casing 14 using an adhesive agent. Thus, the endoscopic capsule 3 forms a capsule having a water-tight structure.

The dome-shaped cover 14a is transparent. An objective lens 15 is attached to a lens frame 16 disposed in the dome-shaped cover 14 at the center of the cylindrical casing 14. The objective lens 15 focuses the light of an image that is incident through the dome-shaped cover 14a. An image sensor (a CCD imager in this embodiment) 17 is disposed at the focusing position of the objective lens 15.

In this embodiment, four white LEDs 18 serving as an illumination system are disposed in the same plane around the objective lens 15. Additionally, for example, a processing circuit 19, a transmission/reception circuit 20, and button-type batteries 21 are disposed on the rear side of the CCD imager 17 in the casing 14. The processing circuit 19 drives the white LEDs 18 to emit light and drives the CCD imager 17 to input a signal of a captured image. The processing circuit 19 then generates an image signal on the basis of the input image capture signal. The transmission/reception circuit 20 transmits the image signal to the extracorporeal device 5 and receives a signal from the extracorporeal device 5. The button-type batteries 21 supply electric power to the processing circuit 19 and the transmission/reception circuit 20.

In addition, a circular coil (circular loop coil) antenna 23 is disposed on the rear end of the button-type battery 21, namely, inside the dome-shaped end of the casing 14 opposite the dome-shaped cover 14a. The antenna 23 is connected to the transmission/reception circuit 20 so as to transmit and receive radio waves. Each of the CCD imager 17, the white LEDs 18, the processing circuit 19, and the transmission/reception circuit 20 is mounted on a substrate (not shown). Each of the substrates is connected to each other using a flexible circuit board.

The processing circuit 19 of the endoscopic capsule 3 generates a control signal to control the timing of an image capture operation of the CCD imager 17. Normally, images are captured at two frames per second. In a part of a body in which the endoscopic capsule 3 moves at a relatively high speed, such as in an esophagus, images are captured at, for example, 15 to 30 frames per second.

The antenna 23 receives a signal transmitted from the extracorporeal device 5. The signal received by the antenna 23 is processed by the transmission/reception circuit 20 and is delivered to the processing circuit 19. The processing circuit 19 controls the timing of an image capture operation of the CCD imager 17 and on/off of illumination of the white LEDs 18 on the basis of the delivered signal. The endoscopic capsule 3 may include a circuit that powers on the endoscopic capsule 3 when a magnetic material, such as a magnet, moves close to the processing circuit 19 of the endoscopic capsule 3, so that the endoscopic capsule 3 is powered on to capture an image before a patient swallows the endoscopic capsule 3.

As shown in Fig. 1A, the antenna unit 4 is attached to the jacket 10 that the patient 2 wears. An enlarged view of the antenna unit 4 is shown in Fig. 3. As shown in Fig. 3, the antenna unit 4 includes antennas 11 a, 11b, ..., and 11i.

The structure of each of transmission/reception parts of the endoscopic capsule 3 and the extracorporeal device 5 is shown in Fig. 4. As shown in Fig. 4, the endoscopic capsule 3 includes an image capturing circuit 31 including the white LEDs 18 and the CCD imager 17. The image capturing circuit 31 captures an image and the processing circuit 19 processes the image to generate a signal. The signal is high-frequency modulated by the transmission/reception circuit 20 and is transmitted from the antenna 23, which is a circular loop antenna.

The signal transmitted from the antenna 23 is received by the antennas 11a, 11b, ..., and 11i of the antenna unit 4 disposed outside the body. The signals received by the antennas 11a, 11 b, ..., and 11 i are demodulated by a transmission/reception circuit 33 and are input to a signal processing circuit 34. The signals input to the signal processing circuit 34 are converted to an image signal. An image is displayed by the liquid crystal monitor 12 on the basis of the image signal. Simultaneously, image data and the like based on the signal input to the signal processing circuit 34 is stored in a memory 35.

Additionally, the image data stored in the memory 35 can be delivered to the liquid crystal monitor 12 in response to the instruction from the user through the operation unit 13. In this way, a previous image can be displayed on the screen of the liquid crystal monitor 12.

In addition, according to the present embodiment, the extracorporeal device 5 includes an antenna location and orientation estimating unit including, for example, a central processing unit (CPU) 36. The CPU 36 serving as the antenna location and orientation estimating unit estimates and computes the location and orientation of the antenna 23 incorporated in the endoscopic capsule 3.

As described below, in this estimation process, an appropriate location and orientation of the antenna 23 are initially determined. Subsequently, the estimation process is repeatedly performed on the basis of the initial location and orientation according to the Gauss-Newton Method until the difference between the estimated values before and after the estimation process becomes less than or equal to a certain small value.

That is, the CPU 36 includes estimating means for performing the estimation process and update correction means for updating and correcting an estimated value so that the difference between the estimated value (i.e., the location and orientation) generated by the estimating means and the value before the estimation is less than or equal to a predetermined value. Note that, as described below, the present invention is not limited to the embodiment that calculates the location and orientation. Alternatively, only one of the location and the orientation may be calculated.

When the user operates the operation unit 13 of the extracorporeal device 5 to input an instruction signal to change the image capturing period or the like into the signal processing circuit 34, the signal processing circuit 34 delivers an instruction signal to the transmission/reception circuit 33. The transmission/reception circuit 33 modulates the instruction signal and transmits that instruction signal from the antennas 11a, 11b, ..., and 11i. The signal transmitted from the antennas 11a, 11b, ... , and 11i is received by the antenna 23 and is demodulated by the transmission/reception circuit 20. Subsequently, for example, the transmission/reception circuit 20 changes the image capturing period in response to the instruction signal, for example.

In the present embodiment, to transmit an image signal captured by the image capturing circuit 31 to the extracorporeal device 5 via the antenna 23 of the endoscopic capsule 3, the endoscopic capsule 3 transmits a reception strength detection signal together with the image signal so that the extracorporeal device 5 can easily detect the reception strength, as shown in Fig. 5A.

That is, each of the frame periods includes a detection period Ta for transmitting the reception strength detection signal and an image signal period Tb for transmitting the image signal. In the detection period Ta, the endoscopic capsule 3 transmits the reception strength detection signal having a predetermined strength (amplitude). In the present embodiment, as shown in Fig. 5B, the image capturing circuit 31 may transmit only the image signal during each of the frame periods.

The reception strength detection signal is received by the antenna 1a, 11 b, ... , and 11 i of the antenna unit 4 and is input to the transmission/reception circuit 33. The transmission/reception circuit 33 demodulates the reception strength detection signal and delivers the demodulated reception strength detection signal to the signal processing circuit 34. The signal processing circuit 34 compares the strengths of the reception strength detection signals received by the antennas 11 s (s = a, b, ... i) with each other. The signal processing circuit 34 then selects the antenna that is optimum for receiving the image signal transmitted by the endoscopic capsule 3 on the basis of the comparison result.

Additionally, the signal processing circuit 34 delivers the image signal received by the optimum antenna and the reception strength detection signals received by the antennas 11s to the memory 35 connected to the signal processing circuit 34. The image signal and the reception strength detection signals are stored (recorded) in the memory 35. The memory 35 is nonvolatile. Examples of the memory 35 include a compact flash (trade name).

In this case, the signal processing circuit 34 may select a plurality of the antennas (e.g., two antennas) for receiving the image signal so as to record two image signals having the same information in the memory 35 at the same time. Additionally, at that time, the signal processing circuit 34 may accumulate the strength of each of the recorded image signals for one frame. Subsequently, the signal processing circuit 34 may hold only the image signal having a greater accumulation value and may delete the other image signal.

In addition, the signal processing circuit 34 delivers the image signal obtained by the optimum antenna to the liquid crystal monitor 12 connected to the signal processing circuit 34. Thus, the image captured by the endoscopic capsule 3 is displayed on the liquid crystal monitor 12.

In the present embodiment, as noted above, the extracorporeal device 5 includes the CPU 36 serving as the antenna location and orientation estimating unit. The antenna location and orientation estimating unit calculates the location and the orientation of the antenna 23 incorporated in the endoscopic capsule 3. As will be described below, the antenna location and orientation estimating unit sets the initial values of the location and orientation of the antenna 23. (For example, the center point of the measurement space and one of the X-axis, Y-axis, and Z-axis directions are determined to be the initial values.)

The CPU 36 estimates detection values of the electromagnetic fields occurring in the antennas 11 a, 11 b, ..., and 11 i disposed outside the body using the initial values (i.e., the zeroth update value). The CPU 36 then obtains the update value for the zeroth location and orientation by computing the sum of squared difference between the estimated value and the actual detected (measured) value. Subsequently, the CPU 36 computes the first location and orientation on the basis of the update values of the zeroth location and orientation. The CPU 36 then repeats a similar estimating operation on the basis of this first location and orientation. If update value after the estimating operation is less than or equal to a sufficiently small value, for example, if the largeness $|\Delta d|$ (= $(\Delta x^2 + \Delta y^2 + \Delta z^2)^{1/2}$) of the update value ($\Delta x$, $\Delta y$, $\Delta z$) for the location is less than or equal to a sufficiently small value, the CPU 36 performs the estimation-value correcting operation so that the update values are defined as the location and orientation of the antenna 23. In this way, the CPU 36 can compute the accurate location and orientation.

The operation of the present embodiment is described below.

In the estimation method according to the present embodiment, the location and orientation of the endoscopic capsule 3 are estimated on the basis of the reception strength detection signals detected using the plurality of antennas 11 a, 11b, ..., and 11 i of the antenna unit 4. This estimation method is described next.

As shown in Fig. 6, in a coordinate system $X_L Y_L Z_L$ based on the antenna 23 which is a circular coil or circular loop and is disposed in the endoscopic capsule 3, the electromagnetic field $H_r$, $H_\theta$, and $E_\varphi$ (including an electrostatic field component, a radiation field component, and an induction field component) at a given point $p(x_L, y_L, z_L)$ is expressed as follows:

$$H_r = (IS/2\pi)(jk/r^2 + 1/r^3)\exp(-jkr)\cos\theta$$

$$H_\theta = (IS/4\pi)(-k^2/r + jk/r^2 + 1/r^3)\exp(-jkr)\sin\theta \qquad (1)$$

$$E_\varphi = -(j\omega\mu IS/4\pi)(jk/r+1/r^2)\exp(-jkr)\sin\theta$$

where $H_r$ and $H_\theta$ denote magnetic field components, $E_\varphi$ denotes an electric field component, I denotes an electric current flowing in the antenna 23, S denote the area of the circular coil of the antenna 23, r denotes the distance between the antenna 23 and the given point P (i.e., r = $(x^2 + y^2 + z^2)^{1/2}$), k = $\omega(\varepsilon\mu)^{1/2}$ ($\varepsilon$ denotes the dielectric constant and $\mu$ denotes the magnetic permeability), and j denotes the imaginary unit.

When the frequency of the electromagnetic field generated by the antenna 23 disposed in the antenna 23 is high and, as shown in Fig. 1A, the distance between the endoscopic capsule 3 and the antennas 11 s disposed on the body surface of the patient 2 is sufficiently large, the radiation field component of the electromagnetic field (electromagnetic wave) that reaches the antennas 11 s becomes the largest. Accordingly, the electrostatic field component and the induction field component are smaller than the radiation field component. Thus, the electrostatic field component and the induction field component can be neglected. Consequently, equations (1) can be rewritten as follows:

$$H_r = 0$$

$$H_\theta = (IS/4\pi)(-k^2/r)\exp(-jkr)\sin\theta \qquad (2)$$

$$E_\varphi = -(j\omega\mu IS/4\pi)(jk/r)\exp(-jkr)\sin\theta$$

Let the antennas 11s disposed on the body surface of the patient 2 be the antenna for detecting the electric field. Then, the equation required for detecting the electric field among equations (2) is equation $E_\varphi$

The electric field $E_\varphi$ in equations (2) represents the radiation electric field, which is considered to be derived from the alternating current theory. Accordingly, the instantaneous value of the electric field $E_\varphi$ can be obtained by multiplying the right-hand side and the left-hand side of equation $E_\varphi$ in equations (2) by $\exp(j\omega t)$ and, subsequently, extracting the real parts as follows:

$$E_\varphi \exp(j\omega t) = -(j\omega\mu IS/4\pi)(jk/r)\exp(-jkr)\sin\theta\exp(j\omega t)$$

$$= (\omega\mu ISk/4\pi r)(\cos U + j\sin U)\sin\theta \qquad (3)$$

where $U = \omega t - kr$.

Here, the real part of equation (3) is extracted. Then, the instantaneous value $E'_\varphi$ of the electric field is expressed as follows:

$$E'_\varphi = (\omega\mu ISk/4\pi r)\cos U\sin\theta \qquad (4)$$

When, as shown in Fig. 7, equation (4) is changed from the polar coordinate system $(r, \theta, \varphi)$ to the Cartesian coordinate system $(X_L, Y_L, Z_L)$, the electric field components $E_{Lx}$, $E_{Ly}$, and $E_{Lz}$ of $X_L$, $Y_L$, and $Z_L$ are expressed as follows:

$$E_{Lx} = E'_\varphi\sin\varphi = (\omega\mu ISk/4\pi r^2)\cos U\cdot(-y_L)$$

$$E_{Ly} = E'_\varphi\cos\varphi = (\omega\mu ISk/4\pi r^2)\cos U\cdot x_L \qquad (5)$$

$$E_{Lz} = 0$$

As shown in Fig. 8, when the electromagnetic waves propagate in a medium, the energy of the electromagnetic waves is absorbed by the medium due to the characteristics (e.g., the electrical conductivity) of the medium. For example, when the electromagnetic waves propagate in the x direction, the exponential decay of the electromagnetic waves due to the attenuation factor $\alpha_d$ can be expressed as follows:

$$A_r = \exp(-\alpha_d r) \qquad (6)$$

$$\alpha_d = (\omega^2\varepsilon\mu/2)^{1/2}[(1 + \kappa^2/(\omega^2\varepsilon^2))^{1/2} - 1]^{1/2}$$

where $\varepsilon = \varepsilon_o\varepsilon_r$ ($\varepsilon_o$: the dielectric constant of vacuum, $\varepsilon_r$: the dielectric constant of the medium), $\mu = \mu_o\mu_r$ ($\mu_o$: the magnetic permeability of vacuum, $\mu_r$: the magnetic permeability of the medium), $\omega$ denotes the angular frequency, and $\kappa$ denotes the electrical conductivity.

Accordingly, the instantaneous value $E_L$ of the electric field when taking into account the characteristics inside the living body is expressed as follows:

$$E_{Lx} = A_r E'_\varphi\sin\varphi = \exp(-\alpha_d r)(\omega\mu ISk/4\pi r^2)\cos U\cdot(-y_L)$$

$$E_{Ly} = A_r E'_\varphi\cos\varphi = \exp(-\alpha_d r)(\omega\mu ISk/4\pi r^2)\cos U\cdot x_L \qquad (7)$$

$$E_{Lz} = 0$$

In addition, the point $P(x_L, y_L, z_L)$ in the Cartesian coordinate system $X_L Y_L Z_L$ based on the antenna 23 of the endoscopic capsule 3 can be converted to that in the coordinate system $X_W Y_W Z_W$ based on the body of the patient 2 as follows:

$$\begin{pmatrix} x_{LP} \\ y_{LP} \\ z_{LP} \end{pmatrix} = R^{-1} \left[ \begin{pmatrix} x_{WP} \\ y_{WP} \\ z_{WP} \end{pmatrix} - \begin{pmatrix} x_{WG} \\ y_{WG} \\ z_{WG} \end{pmatrix} \right] = \begin{pmatrix} R_{00} & R_{01} & R_{02} \\ R_{10} & R_{11} & R_{12} \\ R_{20} & R_{21} & R_{22} \end{pmatrix} \left[ \begin{pmatrix} x_{WP} \\ y_{WP} \\ z_{WP} \end{pmatrix} - \begin{pmatrix} x_{WG} \\ y_{WG} \\ z_{WG} \end{pmatrix} \right] \qquad (8)$$

where $(x_{WP}, y_{WP}, z_{WP})$ and $(x_{WG}, y_{WG}, z_{WG})$ denote the point P and the location of the antenna 23 in the coordinate system $X_W Y_W Z_W$, respectively. In addition, R in the first term on the right-hand side of equation (8) denotes the rotation matrix between the coordinate system $X_W Y_W Z_W$ and the coordinate system $X_L Y_L Z_L$. R can be obtained by using the following formula:

$$\begin{pmatrix} R_{00} & R_{10} & R_{20} \\ R_{01} & R_{11} & R_{21} \\ R_{02} & R_{12} & R_{22} \end{pmatrix} = \begin{pmatrix} \cos\alpha\cos\beta & -\sin\alpha & \cos\alpha\sin\beta \\ \sin\alpha\cos\beta & \cos\alpha & \sin\alpha\sin\beta \\ -\sin\beta & 0 & \cos\beta \end{pmatrix} \qquad (9)$$

where $\alpha$ and $\beta$ denote the amounts of rotation in the polar coordinate system.

Accordingly, the electric field $E_W$ at the given point $(x_{WP}, y_{WP}, z_{WP})$ in the coordinate system $X_W Y_W Z_W$ based on the body of the patient 2 is expressed as follows:

$$\begin{pmatrix} E_{Wx} \\ E_{Wy} \\ E_{Wz} \end{pmatrix} = R \begin{pmatrix} E_{Lx} \\ E_{Ly} \\ E_{Lz} \end{pmatrix} = \begin{pmatrix} R_{00} & R_{10} & R_{20} \\ R_{01} & R_{11} & R_{21} \\ R_{02} & R_{12} & R_{22} \end{pmatrix} \begin{pmatrix} E_{Lx} \\ E_{Ly} \\ E_{Lz} \end{pmatrix} \qquad (10)$$

Thus, by substituting equations (7), (8), and (9) into equation (10), the following equation for the electric field $E_W$ can be obtained:

$$\begin{pmatrix} E_{Wx} \\ E_{Wy} \\ E_{Wz} \end{pmatrix} = \frac{k_t}{r^2} e^{-\alpha_d r} \begin{pmatrix} 0 & (z_{WP} - z_{WG}) & -(y_{WP} - y_{WG}) \\ -(z_{WP} - z_{WG}) & 0 & (x_{WP} - x_{WG}) \\ (y_{WP} - y_{WG}) & -(x_{WP} - x_{WG}) & 0 \end{pmatrix} \begin{pmatrix} g_x \\ g_y \\ g_z \end{pmatrix} \qquad (11)$$

where $k_1$ represents a constant and $(g_x, g_y, g_z)$ represents the orientation of the antenna 23.

When the electric field $E_W$ generated by the antenna 23 is received by, for example, the antenna 11 a of the antenna unit 4 (e.g., a bar antenna shown in Fig. 9, that is, a dipole antenna), the detected electromotive force Va can be computed using the following equation:

$$Va = k_2 E_W \cos\gamma = k_2((E_{Wx} D_{xa} + E_{Wy} D_{ya} + E_{Wz} D_{za}) \qquad (12)$$

where $k_2$ is a constant and Da (see Fig. 9) represents the orientation $(D_{xa}, D_{ya}, D_{za})$ of the antenna 11a of the antenna unit 4 in the coordinate system based on the body of the patient.

As shown in Fig. 3, the plurality of antennas 11 s of the antenna unit 4 are disposed on the body of the patient, and the location and orientation of the antenna 23 is obtained by using an iterative refinement method (the Gauss-Newton method is used in this embodiment). Let x denote the parameter of the location $(x_{WG}, y_{WG}, z_{WG})$ and the orientation $(g_x, g_y, g_z)$ of the antenna 23. The initial value of the parameter is $x^{(0)}$.

Suppose that the kth-order estimation value $x^{(k)}$ is obtained by using the iterative refinement method. When expanding a model function V(x) of the electromotive force generated by the coils of the antennas 11s into a Taylor expansion around $x^{(k)}$, the first-order approximation is:

$$V(x) = V(x^{(k)}) + \left[\frac{\partial V(x)}{\partial x}\right]_{x=x^{(k)}} (x - x^{(k)}) \qquad (13)$$

At that time, let Vm denote the electromotive force measured by the coils of the antennas 11s. Then, the observation equation is expressed as follows:

$$Vm \cong V(x^{(k)}) + \left[\frac{\partial V(x)}{\partial x}\right]_{x=x^{(k)}} (x - x^{(k)}) \quad \text{error } \sigma \qquad (14)$$

where this approximation includes an error σ.

Here, when the first term on the right-hand side of equation (8) is moved to the left-hand side of equation (8), the following expression is obtained:

$$\Delta Vm^{(k)} \cong A^{(k)} \Delta x^{(k)} \text{ error } \sigma \qquad (15)$$

where

$$\Delta Vm^{(k)} = Vm - V(x^{(k)}) = Vm - Vm^{(k)}, \qquad (16)$$

$$\Delta x^{(k)} = x - x^{(k)}, \qquad (17)$$

$$A_{js} = [\partial V_j(x)/\partial x_s]_{x=x}^{(k)} \text{ (j=1 - n and s = 1 - i)} \qquad (18)$$

(row direction: unknown number n, column direction: the number of coils i of the antennas 11 s).

By using equation (18), the solution $\Delta x^{(k)}$ can be expressed as:

$$\Delta x^{(k)} = (A^{t(k)} W A^{(k)})^{-1} A^{t(k)} W \Delta Vm^{(k)} \qquad (19)$$

where $A^t$ is the transposed matrix of A and W is the weighting matrix.

Accordingly, the estimation value of the parameter refined using equation (14) can be obtained as follows:

$$x^{(k+1)} = x^{(k)} + \Delta x^{(k)} \qquad (20)$$

As shown in Fig. 3, when the nine antennas 11a, 11 b, ..., and 11 i are disposed on the body of the patient 2, the matrix A is expressed as follows:

$$A = \begin{bmatrix} \dfrac{\partial V_a}{x_{Wg}} & \dfrac{\partial V_a}{y_{Wg}} & \dfrac{\partial V_a}{z_{Wg}} & \dfrac{\partial V_a}{g_x} & \dfrac{\partial V_a}{g_y} & \dfrac{\partial V_a}{g_z} \\[2mm] \dfrac{\partial V_b}{x_{Wg}} & \dfrac{\partial V_b}{y_{Wg}} & \dfrac{\partial V_b}{z_{Wg}} & \dfrac{\partial V_b}{g_x} & \dfrac{\partial V_b}{g_g} & \dfrac{\partial V_b}{g_z} \\[2mm] M & M & M & M & M & M \\[2mm] \dfrac{\partial V_i}{x_{Wg}} & \dfrac{\partial V_i}{y_{Wg}} & \dfrac{\partial V_i}{z_{Wg}} & \dfrac{\partial V_i}{g_x} & \dfrac{\partial V_i}{g_g} & \dfrac{\partial V_i}{g_z} \end{bmatrix} \qquad (21)$$

The weighting matrix W is:

$$W = \begin{bmatrix} \sigma_0^2 & 0 & 0 & \Lambda & 0 \\ 0 & \sigma_1^2 & 0 & \Lambda & 0 \\ 0 & 0 & \sigma_2^2 & \Lambda & 0 \\ M & M & M & O & M \\ 0 & 0 & 0 & \Lambda & \sigma_\theta^2 \end{bmatrix} \qquad (22)$$

where $\sigma_j$ ($j = 0, 1, \dots 8$) of the weighting matrix W represents the amount of change in the measured voltage of the antennas 11, for example, the environmental noise.

Additionally, the kth $\Delta Vm$ is represented as:

$$\Delta Vm = \begin{bmatrix} Vm_a - V_a(x^{(k)}) \\ Vm_b - V_b(x^{(k)}) \\ Vm_c - V_c(x^{(k)}) \\ M \\ Vm_i - V_i(x^{(k)}) \end{bmatrix} \qquad (23)$$

Therefore, the location and the orientation of the antenna 23 in the endoscopic capsule 3 can be obtained through the following steps (a) through (d).

(a) Set k = 0. Set the initial value of the location of the antenna 23 to $(x_{Wg}^{(0)}, y_{Wg}^{(0)}, z_{Wg}^{(0)})$. Set the initial value of the orientation of the antenna 23 to $(g_x^{(0)}, g_y^{(0)}, g_z^{(0)})$. For example, the initial value of the location is set to the center point of the space in which the antenna 23 is measured. The initial value of the orientation is set to a vector (0, 0, 1) (i.e., the Z-axis direction).
(b) Compute the kth matrix using equations (21), (22), and (23).
(c) Compute the kth amount of update $\Delta x^{(k)}$ using equation (19).
(d) Repeat steps (b) to (d) until the amount of update $\Delta x^{(k)}$ becomes a sufficiently small value.

By performing such an estimation process, the location and the orientation can be accurately estimated (computed).
This procedure of the estimation process is shown in Fig. 10.
As shown in step S1, the CPU 36 sets the initial values of the location and orientation of the antenna 23. In addition, the CPU 36 sets a parameter k to zero (i.e., k = 0) and sets the frame number Nf of an image captured by the endoscopic capsule 3 to one (i.e., Nf =1), where the parameter k represents the kth estimation process of the location and orientation of the antenna 23. The CPU 36 stores, in the memory 35 or the like, the positional information about the antennas 11 a, 11 b, ... and 11 i of the antenna unit 4 removably attached to the body surface of the patient 2.

In step S2, as described in step (b), by using the electromotive force Vm for a frame F1 of the first image obtained through the antennas 11s, the CPU 3 6 computes the matrix A, the weighting matrix W, and the update value matrix ΔVm for an electromotive force, and performs the estimation process of the update value $Ax^{(k)}$ according to equation (19) (the kth estimation process).

In the next step S3, the CPU 36 determines whether, for example, the absolute value of the computed update value $\Delta x^{(k)}$ is less than or equal to a predetermined small value Vth. Note that the value Vth used for the determination may be changed depending on the location and the orientation.

If this condition is not satisfied, the CPU 36, as shown in step S4, increments the parameter k by one. The processing then returns to step S2. Subsequently, the CPU 36 repeats the estimation process until the condition in step S3 is satisfied.

In step S3, if the CPU 36 obtains the update value $\Delta x^{(k)}$ that satisfies the condition, the CPU 36, in step S5, stores the location and the orientation of the antenna 23 in the case of that parameter k in the memory 35 in association with the frame number Nf. Note that the location and the orientation of the antenna 23 are represented as "location information of antenna" in the figure.

In addition, the endoscopic capsule 3 may record the data of the capture time together with the frame number Nf and transmit that data. Also, the extracorporeal device 5 may store the data of signal reception time in the memory. If the capture time is almost the same as the transmission time, one of the capture time and the transmission time may be stored. The coarse (local) moving speed of the endoscopic capsule 3 can be detected using this time information. This moving speed may be used for the estimation of the location.

In the next step S6, the CPU 36 increments the frame number Nf by one. After the CPU 36 sets the initial values of the location and the orientation of the antenna 23 to those obtained in step S5, the processing then returns to step S2. Subsequently, a similar processing is repeated using the electromotive force Vm for the next frame.

In this way, the memory 35 of the extracorporeal device 5 sequentially stores the image data captured by the endoscopic capsule 3, the frame number Nf of the image data, and the information about the location and orientation of the antenna 23. The moving trajectory of the antenna 23 in the living body can be estimated (computed) on the basis of the sequentially stored locations of the antenna 23. The location of the antenna 23 can be considered to be the location of the endoscopic capsule 3. Thus, the information for estimating the moving trajectory of the endoscopic capsule 3 in the living body is stored in the memory 35.

Accordingly, as shown in Fig. 1B, when the extracorporeal device 5 is connected to the cradle 6, the image data, the frame numbers Nf, and the information about the locations and the orientations of the antenna 23 stored in the memory 35 are transferred to the data station 7. Thus, the data station 7 can display such information on the monitor unit 8c.

Figs. 11A and 11B illustrate examples of a display screen on the monitor unit 8c. In Fig. 11A, the trajectory of the endoscopic capsule 3 moving in the body cavity is displayed in the left section of the screen of the monitor unit 8c. The time-series locations of the endoscopic capsule 3 in the body cavity estimated by the extracorporeal device 5 are connected by line. In contrast, the captured image is displayed in the right section of the screen of the monitor unit 8c. For example, the captured image at an estimated position Pi specified by means of a cursor in the left section is displayed.

In the left section of the screen, the reference symbols A, B, and C shown at the right of the trajectory generated by the estimated locations indicate rough locations of body parts. More specifically, the reference symbols A, B, and C indicate an esophagus, an intestinum tenue, and an intestinum crassum, respectively.

In place of the display method shown in Fig. 1A, the display method shown in Fig. 11B may be employed. In this case, an interpolation method, such as a spline method, is used for two adjacent locations. Thus, the locations of the endoscopic capsule 3 for the frames are connected using a smooth curve and are displayed.

As noted above, the monitor unit 8c displays the locations of the endoscopic capsule 3 in the body cavity estimated by the extracorporeal device 5 and the image captured at one of the locations. Accordingly, a user can easily identify at which location in the body cavity the image is captured by the endoscopic capsule 3. As a result, the user can efficiently make a diagnosis.

In addition, if the part that could be a lesioned part is found after the examination of the captured image and needs to be examined more carefully using an endoscope, the location of the part can be accurately estimated. Therefore, that part is identified in a short time without much difficulty. As a result, necessary reexamination and treatment can be efficiently performed.

Accordingly, the present invention can provide the following advantages.

According to the present embodiment, the location of the endoscopic capsule 3 in the body cavity can be precisely estimated. In addition, since, when the electromagnetic waves propagate in the living body, the location and orientation of the endoscopic capsule 3 is estimated using a formulated electric field equation that takes into account the effect of absorption of the electromagnetic energy, the location and orientation of the endoscopic capsule 3 can be highly precisely estimated or computed.

Furthermore, according to the present embodiment, the trajectory of movement of the endoscopic capsule 3 is computed on the basis of the locations estimated by the extracorporeal device 5 and is displayed on the screen. Accordingly, the user can easily identify at which location in the body part in the body cavity the image is captured by the endoscopic capsule 3. As a result, the user can efficiently make a diagnosis of a lesioned part. If the further diagnosis is required, the detailed diagnosis or examination can be efficiently made.

In addition, according to the present embodiment, a bar antenna that detects only an electric field component of the electromagnetic field generated by the antenna 23 disposed in the endoscopic capsule 3 serving as a capsule intracorporeal device is employed. Accordingly, the process of estimating the location and orientation of the antenna 23 (or the endoscopic capsule 3) can be easily carried out, compared with the case where an antenna that detects the electric field and the magnetic field is used.

The typical data about the shapes of body parts, such as an esophagus, a belly, an intestinum crassum, and an intestinum tenue, may be recorded in, for example, the memory 35 so that the user can easily compare the displayed data of the body parts with these data.

While the foregoing description has been made with reference to the estimation process of the location and the orientation of the antenna 23 (or the endoscopic capsule 3), one of the location and the orientation may be estimated (computed). That is, as a modification of the first exemplary embodiment, one of the location and the orientation of the antenna 23 (or the endoscopic capsule 3) may be estimated (computed).

Even in this case, the location or the orientation can be accurately computed using the above-described method. In this case, since the amount of computation is decreased compared with the case where both location and orientation are computed, the location or the orientation can be computed at high speed. Accordingly, in both first exemplary embodiment and the modification thereof, the location and/or the orientation of the antenna 23 (or the endoscopic capsule 3) may be estimated (computed). In such a case, the CPU 36 shown in Fig. 4 functions as an antenna location and/or orientation estimating unit.

It should be noted that, while both the location and orientation are estimated in the following exemplary embodiments, the location and/or the orientation may be computed.

Second Embodiment

A second embodiment of the present invention is described next. The hardware configuration of the second embodiment is similar to that of the first exemplary embodiment. In the second embodiment, an electric field is used that takes into account the case where the endoscopic capsule 3 moves close to the antennas 11 s attached to the body surface of the patient 2.

The operation of the present embodiment is described next.

When the frequency of electromagnetic field generated by the antenna 23 disposed in the endoscopic capsule 3 is high and, as shown in Fig. 1A, the distance between the endoscopic capsule 3 and the antennas 11s attached to the body surface of the patient 2 is sufficiently large, the radiation field component of the electromagnetic field that reaches the antenna 11s becomes the highest. However, if the endoscopic capsule 3 moves close to the antennas 11s, that is, if the distance between the endoscopic capsule 3 and the antennas 11s becomes small, the effect of the induction field becomes large, and therefore, the effect of the induction field cannot be neglected.

Accordingly, in equations (1), only the effect of the electrostatic field is neglected (i.e., the radiation field component and the induction field component are left). Thus, equations (1) are rewritten as follows:

$$H_r = (IS/2\pi)(jk/r^2)\exp(-jkr)\cos\theta$$

$$H_\theta = (IS/4\pi)(-k^2/r + jk/r^2)\exp(-jkr)\sin\theta \qquad (24)$$

$$E_\varphi = -(j\omega\mu IS/4\pi)(jk/r+1/r^2)\exp(-jkr)\sin\theta$$

If the antennas 11 s attached to the body surface of the patient 2 are antennas to detect the electric field, only the equation relating to the electric field $E_\varphi$ is required among equations (24), since no magnetic field components are detected. The electric field $E_\varphi$ represents the radiation electromagnetic field component and the induction field component. This electric field $E_\varphi$ is considered to be derived from the alternating current theory.

Accordingly, the instantaneous value of the electric field $E_\varphi$ can be obtained by multiplying the right-hand side and the left-hand side of equation $E_\varphi$ in equations (24) by $\exp(j\omega t)$ and extracting the real parts as follows:

$$E_\varphi exp(j\omega t)$$

$$= -(j\omega\mu IS/4\pi)(jk/r + 1/r^2)exp(-jkr)sin\theta exp(j\omega t)$$

$$= (\omega\mu ISk/4\pi r^2)\{sinU + RcosU - j(cosU - RsinU)\}sin\theta \qquad (25)$$

where U = ωt - kr and R = kr.

Here, the real parts of equation (25) are extracted. Thus, the instantaneous value of the electric field $E'_\varphi$ can be expressed as follows:

$$E'_\varphi = (\omega\mu ISk/4\pi r^2)\{sinU + RcosU\}sin\theta \qquad (26)$$

Additionally, when, as shown in Fig. 7, equation (26) is changed from the polar coordinate system (r, θ, φ) to the Cartesian coordinate system ($X_L$, $Y_L$, $Z_L$), the electric field components $E_{Lx}$, $E_{Ly}$, and $E_{Lz}$ of $X_L$, $Y_L$, and $Z_L$ are expressed as follows:

$$E_{Lx} = E'_\varphi sin\varphi = (\omega\mu ISk/4\pi r^3)\{sinU + RcosU\}(-y_L)$$

$$E_{Ly} = E'_\varphi cos\varphi = (\omega\mu ISk/4\pi r^3)\{sinU + RcosU\}x_L \qquad (27)$$

$$E_{Lz} = 0$$

Like the first embodiment, when the electromagnetic waves propagate in the medium of the living body and the attenuation in the medium is taken into account, equations (27) are rewritten as follows:

$$E_{Lx} = exp(-\alpha_d r)(\omega\mu ISk/4\pi r^3)\{sinU + RcosU\}(-y_L)$$

$$E_{Ly} = exp(-\alpha_d r)(\omega\mu ISk/4\pi r^3)\{sinU + RcosU\}x_L \qquad (28)$$

$$E_{Lz} = 0$$

Here, with respect to equations (27) or (28) as described in the first embodiment, an equation representing the electric field $E_W$ at a given point P ($x_{WP}$, $y_{WP}$, $z_{WP}$) in the coordinate system $X_W Y_W Z_W$ based on the body of the patient 2 is obtained by using the rotation matrix R.

Thus, the electric field $E_W$ corresponding to equation (11) in the first embodiment can be obtained. In addition, the electromotive force Va generated when the antenna 11 a formed from a bar antenna shown in Fig. 9 receives the electric field $E_W$ can be represented by equation (12). As in the first embodiment, by applying the Gauss-Newton method using the electromotive force Va computed using equation (12), the location and the orientation of the endoscopic capsule 3 (or the antenna 23 in the endoscopic capsule 3) are accurately computed.

According to the present embodiment, even when the distance between the endoscopic capsule 3 serving as a capsule intracorporeal device and the antennas 11s s attached to the body surface of the patient 2 is small, the location and the orientation of the endoscopic capsule 3 can be accurately estimated (computed) since the electric field equation is formulated that takes into account the effect of the induction field.

Third Embodiment

A third embodiment of the present invention is described below with reference to Fig. 12. The configuration of this embodiment is described first. In the configuration of the endoscopic capsule according to this embodiment, the structure (shape) of the antennas 11 used in the antenna unit 4 differs from that used in the first embodiment.

Accordingly, the equation representing the electric field detected by the antennas 11 is different. More specifically, according to the present embodiment, a partly cut circular antenna 11 (a circular antenna that is not a closed loop) shown in Fig. 12 is employed. The other configurations are similar to those of the first embodiment.

The operation of the present embodiment is described next.

In the first exemplary embodiment, the linear dipole antenna 11 is used to detect the electric field. However, in the present embodiment, an antenna having a circularly curved shape as shown in Fig. 12 is used to detect the electric field generated by the antenna 23 incorporated in the endoscopic capsule 3.

When the circular antenna 11 shown in Fig. 12 is used, equations (1) through (11) shown in the first embodiment can be directly applied. However, equation (12) for computing the electromotive force Va detected by the antenna 11a of the first embodiment is replaced by the following equation:

$$Va = k_3 E \sin\gamma = k_3 E |E_x D| / |E||D| \qquad (29)$$

where $k_3$ is a constant corresponding to the constant $k_2$ in equation (12).

The subsequent operations are similar to those represented by equations (13) through (23) of the first embodiment. Thus, the location and the orientation of the antenna 23 can be accurately estimated. According to the present embodiment, by changing the linear antenna 11 used in the first embodiment to the circular antenna 11, the directivity of the antenna 11 can be reduced.

Accordingly, the present embodiment can provide the following advantages.

When receiving the electric field generated by the antenna 23, the circular antenna can reduce the affect caused by the orientation of the antenna 23 compared with the linear antenna as shown in the second embodiment.

A first modification of the present embodiment is described below. In the foregoing description, the location and the orientation of the antenna 23 incorporated in the endoscopic capsule 3 are estimated on the basis of the electromotive force induced in the plurality of antennas 11 of the antenna unit 4 on the reception side. However, in the first modification, the location and the orientation of the antenna 23 are estimated on the basis of the actually detected power (electric power) induced by the antennas 11.

The power detected by the antennas 11 of this modification is expressed as follows:

$$Va^2 = k_3{}^2 E^2 \sin^2\gamma = k_3{}^2 E^2 |E \times D|^2 / |E|^2 |D|^2$$
$$= k_3{}^2 |E \times D|^2 \qquad (30)$$

Equation (30) is a representation that is simpler than equation (29). Thus, it is easy to partial-differentiate equation (30). By estimating the location of the antenna 23 using equation (30), the estimation process can be advantageously speeded up. A second modification of the present embodiment is described below.

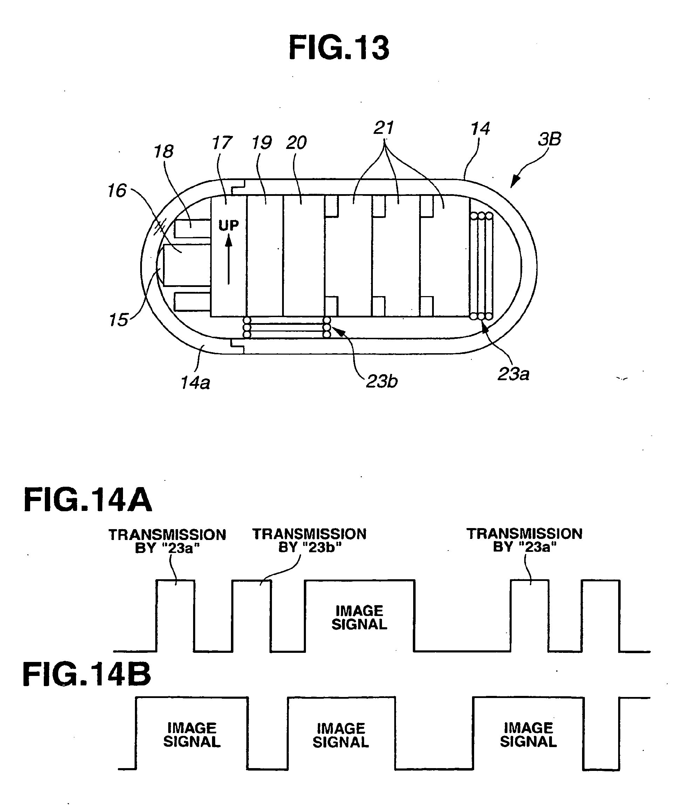

Fig. 13 illustrates an endoscopic capsule 3B according to the second modification of the present embodiment. The endoscopic capsule 3B has a structure similar to the endoscopic capsule 3 shown in Fig. 2. However, the endoscopic capsule 3B includes a circular coil 23a forming the antenna 23 and, for example, a circular coil 23b functioning as a second antenna having an axis direction that is perpendicular to the axis direction of the antenna 23. Note that the axis direction is defined as a direction perpendicular to the plane of a circular coil. For example, the antenna 23 is disposed so that the axis direction of the circular coil 23a substantially coincides with the direction of the center axis of the endoscopic capsule 3B.

The circular coil 23b is disposed in the casing 14 so that the axis direction of the circular coil 23b coincides with a predetermined direction related to the image capturing plane of the CCD imager 17, more specifically, the upward-downward direction of the image capturing plane. In Fig. 13, the upward direction is represented by "Up".

It should be noted that, from the information about the axis direction of the circular coil 23b alone, it cannot be determined whether the axis direction of the circular coil 23b is the upward direction or the downward direction of the image capturing plane of the CCD imager 17. However, by referring to the position information about the circular coils 23a and 23b, the direction of the image capturing plane can be determined. That is, by referring to the position information about the circular coils 23a and 23b, the amount of rotation or rotation angle (from a reference angle) about an axis of the endoscopic capsule 3B in the length direction can be detected. In this case, for example, as shown in Fig. 14A, the circular coils 23a and 23b transmit signals used for estimating the location and the orientation (direction) first. Thereafter, for example, the circular coil 23a transmits an image signal.

Meanwhile, the extracorporeal device 5 selects one of the antennas that is suitable for receiving the image signal on the basis of the signal strengths received from the two circular coils 23a and 23b.

By using the two circular coils 23a and 23b that are mutually perpendicular, the extracorporeal device 5 can accurately compute the position and the orientation of the endoscopic capsule 3B. The estimation process of the positions and orientations of the circular coils 23a and 23b can be carried out in the same way as in the estimation process for one circular coil 23a.

In addition, the extracorporeal device 5 can determine the upward direction of the image capturing plane on the basis of the information about the positions and orientations of the two circular coils 23a and 23b according to this modification and, in particular, the information about the axis direction of the circular coil 23b. The extracorporeal device 5 stores this information in association with the image. Thus, the extracorporeal device 5 displays the captured images so that, for example, the upward direction of the image capturing plane always coincides with the upward direction of the screen.

For example, when the endoscopic capsule 3B moves in an esophagus and rotates about the axis direction thereof relative to the antennas 11 removably attached to the body surface of the patient, the captured image also rotates. However, in this modification, the extracorporeal device 5 can detect, for example, the upward direction of the image capturing plane. Accordingly, even when the image captured by the endoscopic capsule 3B has been rotated, as described above, the extracorporeal device 5 can perform control so that the image is displayed as if the rotation were absent.

That is, when displaying the image captured by the endoscopic capsule 3B, the extracorporeal device 5 can perform control so that the rotation angle of the image about the axis of the endoscopic capsule 3B in the length direction thereof is constant. In this way, even when the endoscopic capsule 3B moves in a human body while rotating about its axis, as described above, the captured images are displayed so that the orientations of the captured images are aligned. Therefore, the user can obtain the images that are easily viewable or diagnosable.

As noted above, according to this modification, an image that helps the user easily diagnose the patient can be displayed. In this modification, after the circular coils 23a and 23b transmit the signals indicating the locations and the orientations thereof, the circular coil 23a transmits the image signal, as shown in Fig. 14A. Alternatively, as shown in Fig. 14B, the circular coils 23a and 23b may alternately transmit the image signals or the circular coils 23a and 23b may transmit the same image signal twice. At that time, the estimation process of the locations and orientations may be carried out.

In addition, this modification can be applied to the first exemplary embodiment or the second exemplary embodiment. Moreover, any combination of part of the above-described embodiments is encompassed by the present invention. Furthermore, while the above-described embodiments have been described with reference to the case where image information optically captured in a body cavity is acquired as the biological information in a human body, the present invention is not limited thereto. For example, the present invention is applicable to capsule medical systems that include a pH sensor and compute the pH information. Furthermore, the endoscopic capsule may include medicinal solution and treatment means for spraying the medicinal solution so as to allow the user to perform medical treatment.

It should be understood that the present invention is not limited to those precise embodiments, and various changes and modifications thereof could be made by one skilled in the art without departing from the invention as defined in the appended claims.

Industrial Applicability

A swallowable capsule medical system that captures the image of the interior of a human body in order to acquire biological information is provided. The capsule medical system transmits a radio wave signal from an antenna incorporated therein. The transmitted signal is received by a plurality of antennas disposed outside the human body so that the location in the human body at which the biological information is acquired can be accurately estimated. Consequently, the biological information can be efficiently used for medical diagnosis.

**Claims**

1. A capsule medical system (1) comprising:

   a capsule intracorporeal device (3) adapted to be placed in a living body (2), the capsule intracorporeal device (3) comprising an antenna (23);
   wireless transmitting means (20) for wirelessly transmitting an electromagnetic wave signal from the antenna (23) of the capsule intracorporeal device (3);
   a plurality of extracorporeal antennas (11) adapted to be disposed outside the living body (2);
   estimating means (36) for estimating at least one of the location and the orientation of the antenna (23) on the basis of the electromagnetic wave signal received by the plurality of extracorporeal antennas (11); and
   updating and correcting means for comparing an estimated value computed using the at least one of the estimated location and the estimated orientation with an actually detected value and repeatedly updating and correcting the at least one of the location and the orientation estimated by the estimating means (36) until an update value for the at least one of the location and the orientation computed on the basis of the compared values is less than or equal to a predetermined value, **characterized in that** the estimating means (36) is adapted to estimate the location of the antenna (23) on the basis of the electromagnetic wave signal received by the plurality of extracorporeal antennas (11) using a theoretical formula that takes into account the attenuation of the electromagnetic wave signal in the living body (2).

2. The capsule medical system according to Claim 1, wherein the capsule intracorporeal device (3) comprises biological information acquiring means (15) for acquiring biological information; and the wireless transmitting means (20) transmits the biological information acquired by the biological information acquiring means (15) using the electromagnetic wave signal.

3. The capsule medical system according to Claim 1, further comprising:

trajectory computing means for computing a trajectory of movement of the capsule intracorporeal device (3) in the living body (2) on the basis of the location of the antenna (23) estimated by the updating and correcting means.

4. The capsule medical system according to Claim 1, wherein the plurality of extracorporeal antennas (11) include electric field detection antennas for detecting the electric field component of the electromagnetic wave.

5. The capsule medical system according to Claim 1, wherein the theoretical formula involves an attenuation factor due to which the electromagnetic wave signal exponentially decays when it propagates in the living body (2).


**Patentansprüche**

1. Eingekapseltes medizinisches System (1) mit:

einer eingekapselten intrakorporalen Vorrichtung (3), die in einen lebenden Körper (2) eingebracht zu werden vermag, wobei die eingekapselte intrakorporale Vorrichtung (3) eine Antenne (23) umfasst;
drahtlosen Übertragungsmitteln (20) zum drahtlosen Übertragen eines elektromagnetischen Wellensignals von der Antenne (23) der eingekapselten intrakorporalen Vorrichtung (3);
mehreren extrakorporalen Antennen (11), die außerhalb des lebenden Körpers (2) angeordnet zu werden vermögen;
einem Schätzmittel (36) zum Schätzen der Lage und/oder der Ausrichtung der Antenne (23) auf der Basis des von den mehreren extrakorporalen Antennen (11) empfangenen elektromagnetischen Wellensignals; und
Aktualisierungs- und Korrekturmitteln zum Vergleichen eines Schätzwertes, der mittels der geschätzten Lage und/oder der geschätzten Ausrichtung berechnet wird, mit einem tatsächlich erfassten Wert, und zum wiederholten Aktualisieren und Korrigieren der von dem Schätzmittel (36) geschätzten Lage und/oder Ausrichtung, bis ein aktueller Wert für die auf der Basis der Vergleichswerte berechnete Lage und/oder Ausrichtung kleiner oder gleich einem vorbestimmten Wert ist,
**dadurch gekennzeichnet, dass** das Schätzmittel (36) die Lage der Antenne (23) auf der Basis des von den mehreren extrakorporalen Antennen (11) empfangenen elektromagnetischen Wellensignals unter Anwendung einer theoretischen Formel, welche die Dämpfung des elektromagnetischen Wellensignals in dem lebenden Körper (2) berücksichtigt, zu schätzen vermag.

2. Eingekapseltes medizinisches System nach Anspruch 1, wobei die eingekapselte intrakorporale Vorrichtung (3) ein Mittel (15) zum Erheben biologischer Information umfasst, um biologische Information zu erheben; und das drahtlose Übertragungsmittel (20) unter Verwendung des elektromagnetischen Wellensignals die von dem Mittel (15) zum Erheben biologischer Information erhobene biologische Information überträgt.

3. Eingekapseltes medizinisches System nach Anspruch 1, ferner mit:

einem Bahn-Berechnungsmittel zum Berechnen einer Bewegungsbahn der eingekapselten intrakorporalen Vorrichtung (3) in dem lebenden Körper (2) auf der Basis der von den Aktualisierungs- und Korrekturmitteln geschätzten Lage der Antenne (23).

4. Eingekapseltes medizinisches System nach Anspruch 1, wobei die mehreren extrakorporalen Antennen (11) Antennen zum Erfassen eines elektrischen Feldes aufweisen, um die elektrische Feldkomponente der elektromagnetischen Welle zu erfassen.

**5.** Eingekapseltes medizinisches System nach Anspruch 1, wobei die theoretische Formel einen Dämpfungsfaktor beinhaltet, aufgrund dessen das elektromagnetische Wellensignal exponentiell zerfällt, wenn es sich in dem lebenden Körper (2) ausbreitet.

## Revendications

**1.** Système médical de type capsule (1) comprenant :

un dispositif intracorporel de type capsule (3) adapté à être placé dans un corps vivant (2), le dispositif intracorporel de type capsule (3) comprenant une antenne (23) ;
un moyen de transmission sans fil (20) pour transmettre sans fil un signal d'onde électromagnétique à partir de l'antenne (23) du dispositif intracorporel de type capsule (3) ;
une pluralité d'antennes extracorporelles (11) adaptées à être disposées à l'extérieur du corps vivant (2) ;
un moyen d'estimation (36) pour estimer au moins un de l'emplacement et de l'orientation de l'antenne (23) sur la base du signal d'onde électromagnétique reçu par la pluralité d'antennes extracorporelles (11) ; et
un moyen de mise à jour et de correction pour comparer une valeur estimée calculée en utilisant l'au moins un de l'emplacement estimé et de l'orientation estimée avec une valeur réellement détectée et mettre à jour et corriger de façon répétée l'au moins un de l'emplacement et de l'orientation estimé(e) par le moyen d'estimation (36) jusqu'à ce qu'une valeur de mise à jour pour l'au moins un de l'emplacement et de l'orientation calculée sur la base des valeurs comparées soit inférieure ou égale à une valeur prédéterminée, **caractérisé en que** le moyen d'estimation (36) est adapté à estimer l'emplacement de l'antenne (23) sur la base du signal d'onde électromagnétique reçu par la pluralité d'antennes extracorporelles (11) en utilisant une formule théorique qui prend en compte l'atténuation du signal d'onde électromagnétique dans le corps vivant (2).

**2.** Système médical de type capsule selon la revendication 1, dans lequel le dispositif intracorporel de type capsule (3) comprend un moyen d'acquisition d'informations biologiques (15) pour acquérir des informations biologiques ; et le moyen de transmission sans fil (20) transmet les informations biologiques acquises par le moyen d'acquisition d'informations biologiques (15) en utilisant le signal d'onde électromagnétique.

**3.** Système médical de type capsule selon la revendication 1, comprenant en outre :

un moyen de calcul de trajectoire pour calculer une trajectoire de mouvement du dispositif intracorporel de type capsule (3) dans le corps vivant (2) sur la base de l'emplacement de l'antenne (23) estimé par le moyen de mise à jour et de correction.

**4.** Système médical de type capsule selon la revendication 1, dans lequel la pluralité d'antennes extracorporelles (11) incluent des antennes de détection de champ électrique pour détecter la composante de champ électrique de l'onde électromagnétique.

**5.** Système médical de type capsule selon la revendication 1, dans lequel la formule théorique implique un facteur d'atténuation du fait duquel le signal d'onde électromagnétique diminue de façon exponentielle lorsqu'il se propage dans le corps vivant (2).

# FIG.1A

# FIG.1B

# FIG.2

# FIG.3

# FIG.4

23

20

TRANSMISSION/
RECEPTION
CIRCUIT

19

PROCESSING
CIRCUIT

31

IMAGE
CAPTURING
CIRCUIT

3

5

11a   11b         11i

4

TRANSMISSION/     33
RECEPTION
CIRCUIT

34

SIGNAL
PROCESSING
CIRCUIT

13

OPERATION
UNIT

35

MEMORY

12

36

CPU
(ANTENNA LOCATION
AND ORIENTATION
ESTIMATING UNIT)

1

# FIG.5A

ONE FRAME PERIOD

Ta          Tb

| RECEPTION STRENGTH DETECTION SIGNAL | IMAGE SIGNAL | | RECEPTION |

TIME

# FIG.5B

ONE FRAME PERIOD

Tb

| IMAGE SIGNAL | | IMAGE |

TIME

# FIG.6

# FIG.7

# FIG.8

# FIG.9

# FIG.10

START OF ESTIMATION
PROCESS

S1

SET INITIAL VALUES OF ANTENNA: k=0 AND Nf=1

S2

kTH ESTIMATION PROCESS (COMPUTATION OF
MATRIX A AND UPDATE VALUE Δx$^{(k)}$, ETC.

S3

$|Δx^{(k)}| \leq Vth?$

NO

S4

k=k+1

YES

S5

STORE ANTENNA LOCATION INFORMATION
IN MEMORY TOGETHER WITH Nf

S6

Nf=Nf+1, k=0

S7

SET INITIAL VALUE OF ANTENNA TO LOCATION
INFORMATION STORED AT STEP S5

# FIG.11A

# FIG.11B

A

B

C

# FIG.12

Da

E

11

γ

# FIG.13

# FIG.14A

TRANSMISSION BY "23a"  TRANSMISSION BY "23b"  TRANSMISSION BY "23a"

IMAGE SIGNAL

# FIG.14B

IMAGE SIGNAL  IMAGE SIGNAL  IMAGE SIGNAL

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003135389 A
- JP 3571675 B
- WO 2005067781 A1